(19) Europäisches Patentamt
European Patent Office
Office européen des brevets

(11) Publication number: 0 355 784 A1

# (12) EUROPEAN PATENT APPLICATION

(21) Application number: 89115439.5

(22) Date of filing: 22.08.89

(51) Int. Cl.4: C07C 323/60 , C07D 339/08 , A61K 31/195

(30) Priority: 24.08.88 US 235587

(43) Date of publication of application: 28.02.90 Bulletin 90/09

(84) Designated Contracting States: ES GR

(71) Applicant: SCHERING CORPORATION
2000 Galloping Hill Road
Kenilworth New Jersey 07033(US)

(72) Inventor: Czarniecki, Michael F.
30 Gildersleeve Place
Watchung New Jersey 07060(US)
Inventor: Lehman, Laura S.
8126 Camino Del Sol
La Jolla California 92037(US)

(74) Representative: von Kreisler, Alek et al
Patentanwälte Von Kreisler-Selting-Werner-Schönwald-Fues-Dallmeyer Deichmannhaus
D-5000 Köln 1(DE)

## (54) Mercapto-acylamino acid antihypertensives.

(57) Mercapto-acylamino acids of the formula:

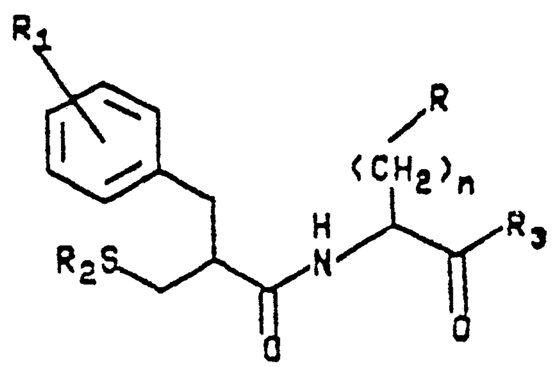

wherein n is 0-4;
R is

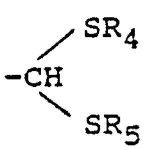 , 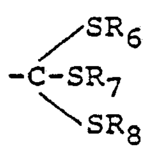 , 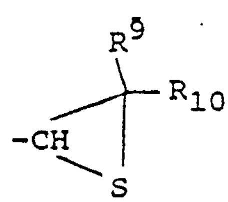 ;

$R_1$ is 1-3 substituents selected from the group consisting of hydrogen, halogeno, lower alkyl, cyclolower alkyl, lower alkoxy, hydroxy, aryl, aryloxy, cyano, aminomethyl, carboxy, lower alkoxy carbonyl and

EP 0 355 784 A1

carbamyl;
$R_2$ is hydrogen,
$R_{11}-\overset{O}{\overset{\|}{C}}-$ or $R_{11}-\overset{O}{\overset{\|}{C}}-OCH_2-$, wherein $R_{11}$ is lower alkyl, aryl or arylmethyl;
$R^3$ is $-OR_{12}$,

$$-\overset{R_{12}}{\overset{|}{N}}R_{13} \quad \text{or} \quad -O\overset{R_{14}}{\overset{|}{C}}H-\underset{\underset{O}{\|}}{C}-\overset{R_{12}}{\overset{|}{N}}R_{13};$$

$R_4$, $R_5$, $R_6$, $R_7$, $R_8$, $R_9$ and $R_{10}$ are independently lower alkyl, aryl lower alkyl, (cyclolower alkyl) lower alkyl, substituted aryl lower alkyl or substituted (cyclolower alkyl) lower alkyl, wherein in the substituents on the aryl and cyclolower alkyl portions are 1-3 substituents selected from the group consisting of lower alkyl, hydroxy, halogeno, lower alkoxy and amino, or $R_4$ and $R_5$ are alkyl and together with the sulfur and carbon atoms to which they are attached form a 5-7 membered ring;
$R_{12}$ and $R_{13}$ are independently hydrogen, lower alkyl or substituted lower alkyl wherein the substituents are selected from the group consisting of 1 or 2 hydroxy groups, 1 or 2 lower alkoxy groups, lower alkoxy lower alkoxy, halogeno, halogeno lower alkoxy, amino, mono- or di-lower alkylamino, aryl, substituted aryl wherein the substituents on aryl are 1-3 substituents selected from the group consisting of lower alkyl, hydroxy, halogeno, lower alkoxy and amino, and a 5-6 membered saturated ring comprising 1-2 oxygen atoms as ring members wherein the ring carbon atoms can be substituted with 0-2 lower alkyl substituents; or $R_{12}$ and $R_{13}$ together with the nitrogen to which they are attached complete a 5-7 membered ring, wherein one of the 4-6 ring members comprising $R_{12}$ and $R_{13}$ may be a nitrogen atom, an alkyl-substituted nitrogen atom or an oxygen atom, and wherein the ring may be substituted on the ring carbon atoms with substituents chosen from alkyl and hydroxy groups;
$R_{14}$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl or carbamoylalkyl;
the pharmaceutically acceptable salts thereof and their combinations with atrial natriuretic factors or angiotensin converting enzyme inhibitors are disclosed. Such preparations are useful for treating hypertension and congestive heart failure.

# MERCAPTO-ACYLAMINO ACID ANTIHYPERTENSIVES

## SUMMARY OF THE INVENTION

The present invention relates to mercapto-acylamino acids useful in the treatment of hypertension and cove heart failure.

The invention also relates to the treatment of hypertension and congestive heart failure with a combination of a mercapto-acylamino acid and an atrial natriuretic factor (ANF) and with a combination of a mercapto-acylamino acid and an angiotensin converting enzyme (ACE) inhibitor.

Other aspects of the invention relate to pharmaceutical compositions comprising a mercapto-acylamino acid of this invention, alone or in combination with an ANF or an ACE inhibitor, and to methods of treatment of hypertension and congestive heart failure comprising administering a mercapto-acylamino acid of this invention, alone or in combination with an ANF or an ACE inhibitor to a mammal in need of such treatment.

## BACKGROUND OF THE INVENTION

Human hypertension represents a disease of multiple etiologies. Included among these is a sodium and volume dependent low renin form of hypertension. Drugs that act to control one aspect of hypertension will not necessarily be effective in controlling another.

A variety of mercapto-acylamino acids are known as enkephalinase inhibitors useful as analgesics and in the treatment of hypertension.

U.S. 4,513,009 to Roques et al discloses, inter alia, compounds of the formula.

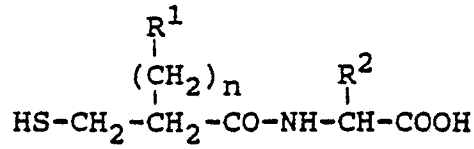

wherein n is 0 to 1; $R^1$ includes hydrogen, optionally substituted alkyl, optionally substituted phenyl, cyclohexyl and thienyl; and $R^2$ includes hydrogen, optionally substituted alkyl, optionally substituted benzyl, phenyl, phenoxyalkyl and optionally substituted mercaptoalkyl. The compounds are disclosed as principally having enkephalinase activity, but also are said to be antihypertensives.

U.S. 4,401,677 to Greenberg et al and EPA 38,046 to Wilkinson disclose compounds of a scope similar to Roques et al, the former disclosing analgesic activity and the latter disclosing a greater specificity for enkephalinase than ACE. U.S. 4,053,651 to Ondetti et al discloses the use of similar compounds in the treatment of renin-angiotensin related hypertension.

It has recently been discovered that the heart secretes a series of peptide hormones called atrial natriuretic factors (ANF) which help to regulate blood pressure, blood volume and the excretion of water, sodium and potassium. ANF were found to produce a short-term reduction in blood pressure and to be useful in the treatment of congestive heart failure. See P. Needleman et al, "Atriopeptin: A Cardiac Hormone Intimately Involved in Fluid, Electrolyte and Blood-Pressure Homeostasis", N. Engl. J. Med., 314, 13 (1986) pp. 828-834, and M. Cantin et al in "The Heart as an Endocrine Gland", Scientific American, 254 (1986) pg. 76-81.

A class of drugs known to be effective in treating some types of hypertension is ACE inhibitors, which compounds are useful in blocking the rise in blood pressure caused by increases in vascular resistance and fluid volume due to the formation of angiotensin II from angiotensin I. For a review of ACE inhibitors, see M. Wyvratt and A. Patchett, "Recent Developments in the Design of Angiotensin Converting Enzyme Inhibitors" in Med. Res. Rev. Vol. 5, No. 4 (1985) pp. 483-531.

## DETAILED DESCRIPTION

The mercapto-acylamino acids of this invention are represented by the following formula:

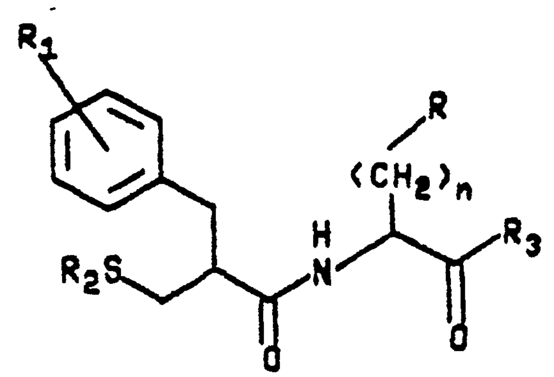

I

wherein n is 0-4;
R is

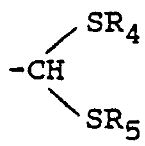
,
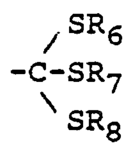
or
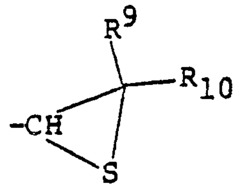
;

$R_1$ is 1-3 substituents selected from the group consisting of hydrogen, halogeno, lower alkyl, cyclolower alkyl, lower alkoxy, hydroxy, aryl, aryloxy, cyano, aminomethyl, carboxy, lower alkoxy carbonyl and carbamyl;
$R^2$ is hydrogen,
$R_{11}-\overset{O}{\overset{\|}{C}}-$ or $R_{11}-\overset{O}{\overset{\|}{C}}-OCH_2-$, wherein $R_{11}$ is lower alkyl, aryl or arylmethyl;
$R_3$ is $-OR_{12}$,

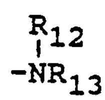
or
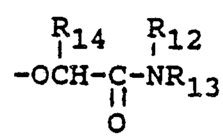

$R_4$, $R_5$, $R_6$, $R_7$, $R_8$, $R_9$ and $R_{10}$ are independently lower alkyl, aryl lower alkyl, (cyclolower alkyl) lower alkyl, substituted aryl lower alkyl or substituted (cyclolower alkyl) lower alkyl, wherein the substituents on the aryl and cyclolower alkyl portions are 1-3 substituents selected from the group consisting of lower alkyl, hydroxy, halogeno, lower alkoxy and amino, or $R_4$ and $R_5$ are alkyl and together with the sulfur and carbon atoms to which they are attached form a 5-7 membered ring;
$R_{12}$ and $R_{13}$ are independently hydrogen, lower alkyl or substituted lower alkyl wherein the substituents are selected from the group consisting of 1 or 2 hydroxy groups, 1 or 2 lower alkoxy groups, lower alkoxy lower alkoxy, halogeno, halogeno lower alkoxy, amino, mono- or di-lower alkylamino, aryl, substituted aryl wherein the substituents on aryl are 1-3 substituents selected from the group consisting of lower alkyl, hydroxy, halogeno, lower alkoxy and amino, and a 5-6 membered saturated ring comprising 1-2 oxygen atoms as ring members wherein the ring carbon atoms can be substituted with 0-2 lower alkyl substituents; or $R_{12}$ and $R_{13}$ together with the nitrogen to which they are attached complete a 5-7 membered ring, wherein one of the 4-6 ring members comprising $R_{12}$ and $R_{13}$ may be a nitrogen atom, an alkyl-substituted nitrogen atom or an oxygen atom, and wherein the ring may be substituted on the ring carbon atoms with substituents chosen from alkyl and hydroxy groups;
$R_{14}$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl or carbamoylalkyl;
and the pharmaceutically acceptable salts thereof.

As used herein, the term "lower alkyl" means straight or branched alkyl chains of 1 to 6 carbon atoms,

and "lower alkoxy" similarly refers to alkoxy groups having 1 to 6 carbon atoms. "Cyclolower alkyl" means cyclic alkyl groups of 3-6 carbon atoms.

"Aryl" means mono-cyclic or fused ring bicyclic carbocyclic aromatic groups having 6 to 10 ring members or mono-cyclic or fused ring bicyclic aromatic groups wherein 1-2 ring members are independently be nitrogen, oxygen or sulfur. "Substituted aryl" are those groups wherein the carbon ring members may be substituted by one to three substituents as defined above. Examples of carbocyclic aryl groups are phenyl, α-naphthyl and β-naphthyl, and examples of heterocyclic aryl groups are furyl, thienyl, benzofuryl, benzothienyl, indolyl and pyridyl. All positional isomers, e.g. 2-pyridyl, 3-pyridyl, are contemplated.

"Halo" refers to fluorine, chlorine, bromine or iodine radicals.

Preferred compounds of formula I are those wherein $R_1$ is hydrogen or o-, m- or p-methyl, methoxy or halogeno. Also preferred are compounds of formula I wherein $R_2$ is hydrogen, acetyl or benzoyl. Another group of preferred compounds of formula I in that wherein $R_3$ is hydroxy, alkoxy, especially methoxy or ethoxy, or amino. Still other preferred compounds of formula I are those wherein $R_4$ and $R_5$ or $R_6$, $R_7$ and $R_8$ are each lower alkyl, especially methyl or wherein $R_9$ and $R_{10}$ are each hydrogen. A preferred value for n is 1.

More preferred are compounds of formula I wherein $R_1$ is hydrogen, methyl, methoxy or halogeno, $R_2$ is hydrogen, acetyl or benzoyl, $R_3$ is hydroxy, alkoxy or amino and n is 1. Of these more preferred compounds, those wherein $R_4$ and $R_5$ or $R_6$, $R_7$ and $R_8$ are each lower alkyl or wherein $R_9$ and $R_{10}$ are each hydrogen are especially preferred. Also especially preferred are compounds of formula I wherein R is

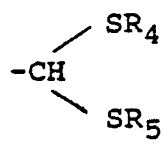 .

A preferred compound of this invention is N-(3-phenyl-2-(mercaptomethyl)propionyl)-(S)-4-(methylmercapto)methionine. The R,S and S,S diastereomers are both preferred.

Compounds of this invention may, depending on the nature of functional groups, form addition salts with various inorganic and organic acids and bases. Such salts include salts prepared with organic and inorganic acids, e.g. HCl, HBr, $H_2SO_4$, $H_3PO_4$, methanesulfonic acid, toluenesulfonic acid, maleic acid, furmaric acid and camphorsulfonic acid. Salts prepared with bases include ammonium salts, alkali metal salts, e.g. sodium and potassium salts, and alkaline earth salts, e.g. calcium and magnesium salts.

The salts may be formed by conventional means, as by reacting the free acid or base forms of the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

Compounds of formula I have two or more asymmetrical carbon atoms and therefore include various stereoisomers. All stereoisomers are included within the scope of the present invention.

Compounds of the present invention may be prepared by using coupling reactions well known in the peptide art. Typically, one can join a 2-acetylthio-3-(substituted)-propionic acid of formula II with an amino acid ester of formula III. The following reaction Scheme is an example:

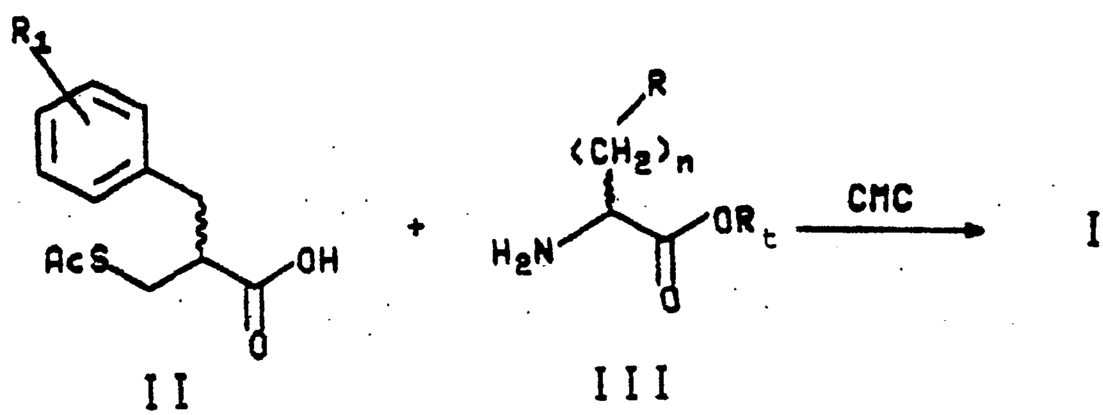


II III I

In the above Scheme 1, n, R and $R_1$ are as defined above, Ac is acetyl and $R_t$ is methyl, ethyl, t-butyl or

aralkyl (e.g. benzyl). In the reaction, an amino acid ester of formula III and a 2-acetylthio propionic acid of formula II are reacted at room temperature in an inert solvent such as methylene chloride in the presence of coupling agents such as 1-cyclohexyl-3-(2-morpholinoethyl)carbodiimide metho-p-toluenesulfonate (CMC) and 1-hydroxybenzotriazole (HOBT) or DEC (1-(3-dimethylaminopropyl)-3-ethylcarbodiimide) or DCC (dicyclohexyl carbodiimide) and HOBT. The resultant isomers are separated by chromatography (e.g. reverse phase high pressure liquid chromatography (HPLC)) and deprotected as desired at the carboxy and mercapto positions.

Alternatively, a propionic acid of formula II may be reacted with thionyl chloride to prepare the corresponding propionyl chloride, which may then be reacted with an amino acid ester of formula III or with the corresponding free acid IIIa in an inert solvent such as acetonitrile in the presence of a base such as triethylamine to give isomers of formula I. The following Scheme 2 is an example:

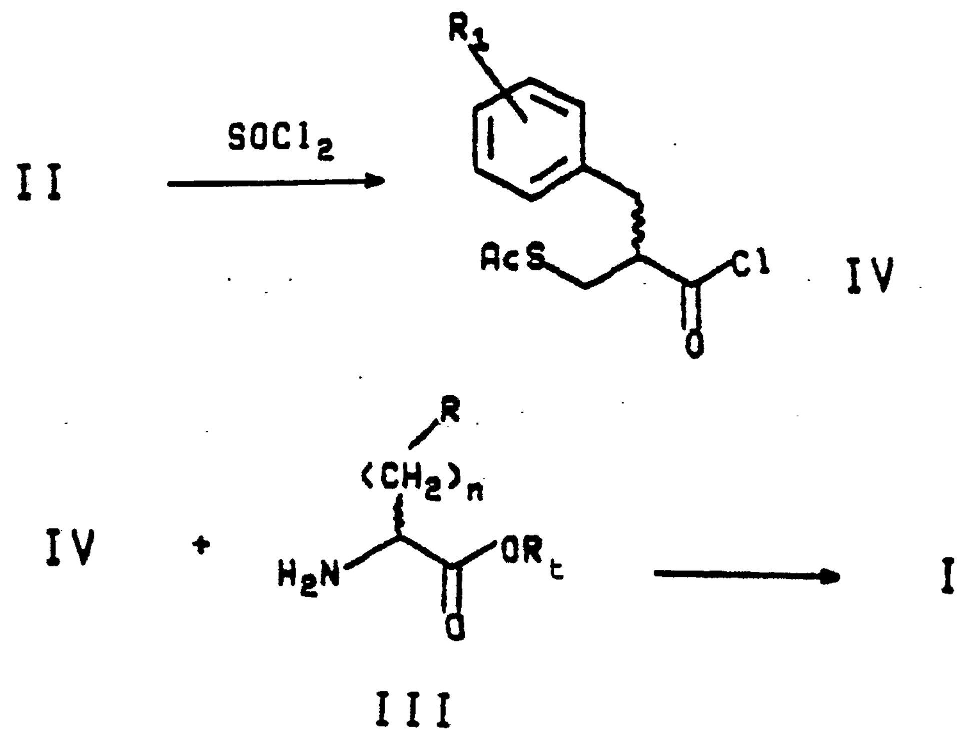

II
IV
III
I

wherein n, Ac, R, $R_1$ and $R_t$ are as defined above, and wherein $R_t$ may also be hydrogen.

Other $R_3$ esters of compounds of formula I are prepared by standard esterification techniques, for example N-(t-butoxycarbonyl)-S-4-(methylmercapto)methionine is reacted with 2-methoxyethanol in the presence of a base such as 4-dimethylaminopyridine, the amino function is deprotected and the resultant methoxyethyl ester is reacted with a compound of formula II in a manner similar to that described in Scheme 2. In another example, N-(t-butoxycarbonyl)-S-4(methylmercapto)methionine is reacted with N,N-diethylbromoacetamide and a reagent such as cesium carbonate, the resultant glycolamide ester is deprotected at the amino function (i.e., the t-butoxycarbonyl group is removed) and a reaction similar to that described in Scheme 2 is carried out.

Compounds of formula I wherein $R_3$ is $-NR_{12}R_{13}$ are prepared by coupling reactions as described above in Schemes 1 and 2 by replacing the amino acid ester III with an amide or substituted amide as shown in Scheme 3:

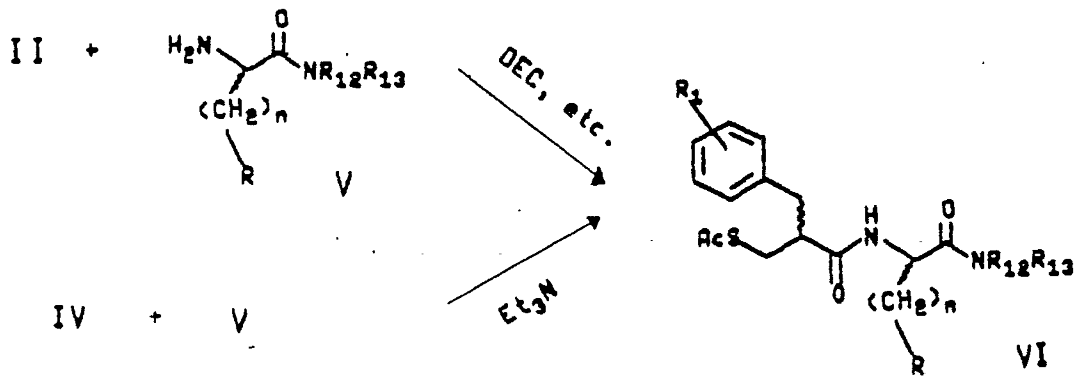


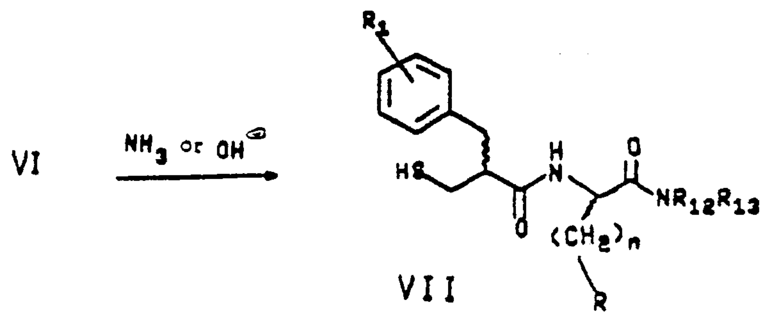


Alternatively, compounds of formula I wherein $R_3$ is $-NR_{12}R_{13}$ may be prepared by coupling a propionyl chloride of formula IV with an amino acid of formula IIIa in the presence of a base to obtain a compound of formula Ia wherein $R_3$ is OH, and then coupling the desired $-NR_{12}R_{13}$ group to the carboxylic group using a typical peptide-coupling reaction. Scheme 4 shows an example of such a procedure:

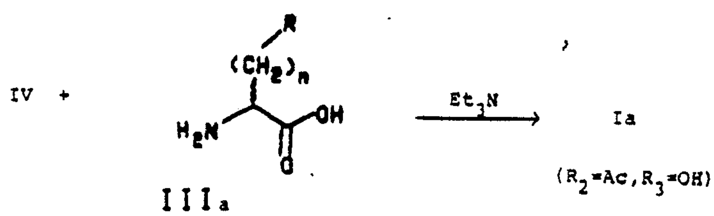


Ia $\xrightarrow[\text{DEC, etc.}]{NHR_{12}R_{13}}$ VI $\xrightarrow{NH_3 \text{ or } OH^-}$ VII

A third method for preparing compounds of formula I wherein $R_3$ is $-NR_{12}R_{13}$ comprises reacting a propionic acid of formula II with an amino acid t-butyl ester of formula III, removing the t-butyl ester and coupling the $-NR_{12}R_{13}$ group to the carboxylic acid group as above.

Compounds wherein $R_2$ is $R_{11}CO$- may be prepared by known methods, for example by adding a mercaptoacid of formula $R_{11}$-COSH to an acrylic acid to obtain a thio-substituted propionic acid analogous to compounds of formula II. Alternatively, an amide of formula I wherein $R_2$ is hydrogen may be reacted with a compound of formulae $R_{11}COCl$ in the presence of a base to obtain the desired sulfur substituted derivative.

Starting materials of formulae II and III are known in the art or may be prepared by methods well known to those skilled in the art from amino acids containing olefin or carboxylic acid side chains. Typical reaction schemes follow.

For compounds of formula III wherein R is

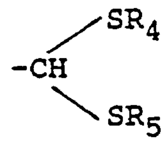
,

an N-protected amino acid with an olefinic side chain can undergo ozonolysis, followed by reduction with dimethyl sulfide (DMS), then treatment with mercaptan ($HSR_3$, $HSR_4$) in the presence of an acid and finally deprotection of the amino group:

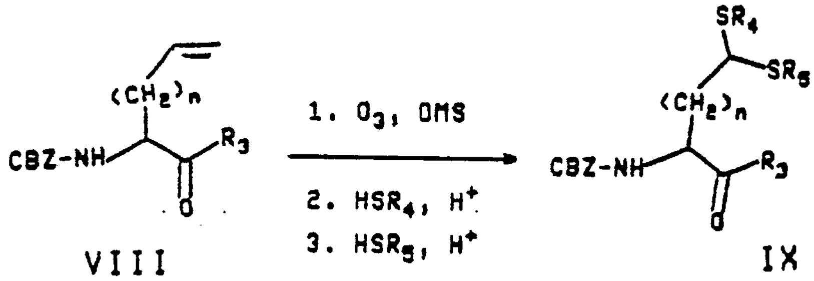


VIII IX

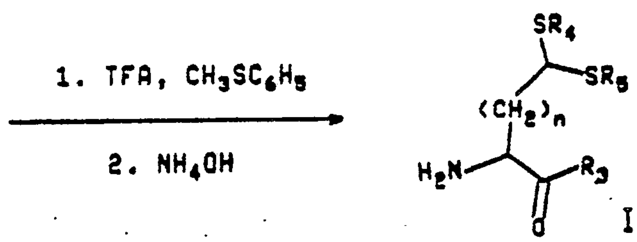


IX IIIb

wherein CBZ is carbobenzyloxy and TFA is trifluoroacetic acid.

For compounds of formula III wherein R is

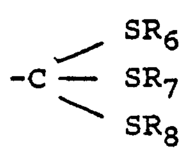
,

an N-protected amino acid with a carboxylic acid side chain can be converted to the acid chloride at the side chain, then successively treated with mercaptans ($HSR_6$, $HSR_7$, $HSR_8$) in the presence of zinc chloride, and finally deprotected at the amino group:

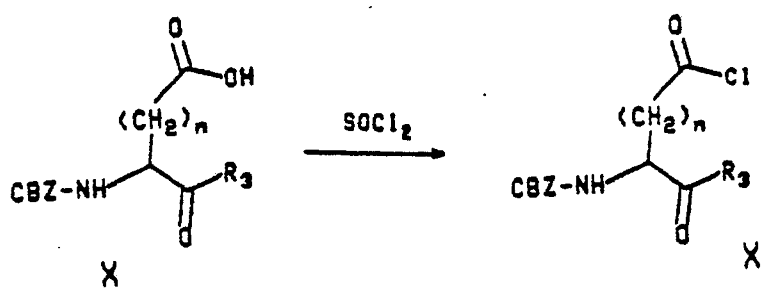


X XI

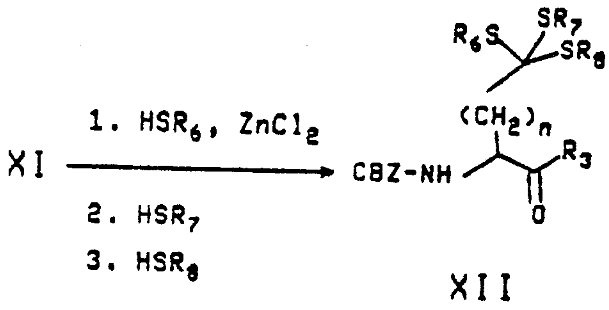


XI → XII

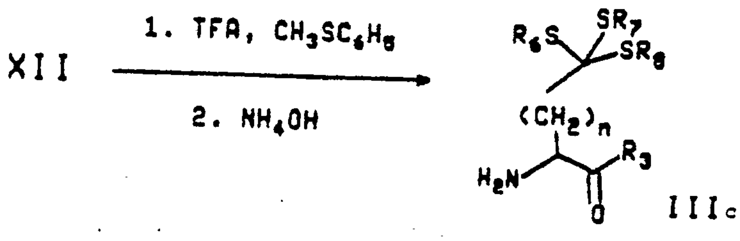


XII → IIIc

For compounds of formula III wherein R is

, an N-protected amino acid with an olefinic side chain can be treated with sulfur monochloride, followed by reduction and dehydrohalogenation (e.g. with sodium sulfate) to form the episulfide, and finally be deprotected at the amino group:

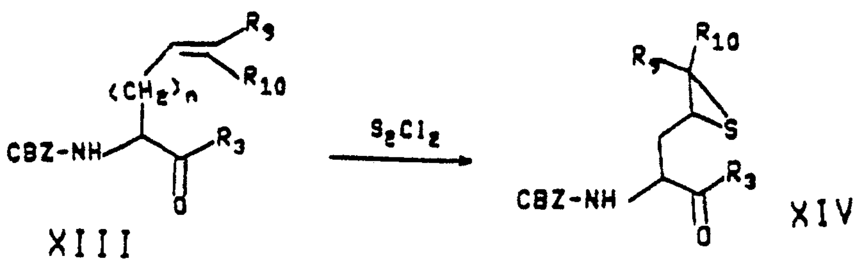


XIII → XIV

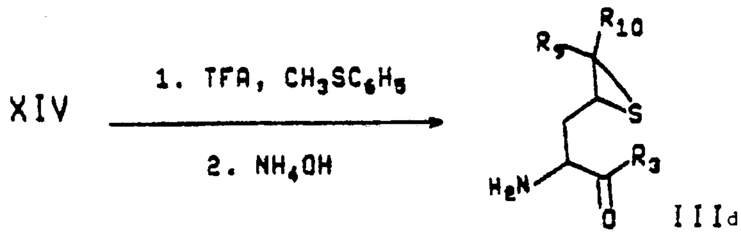


XIV → IIId

A second aspect of the invention is the administration of a combination of an ANF and a compound of formula I for the treatment of hypertension.

As indicated by Needleman et al., a number of ANF have been isolated so far, all having the same core sequence of 17 amino acids within a cysteine disulfide bridge, but having different N-termini lengths. These peptides represent N-terminal truncated fragments (21-48 amino acids) of a common preprohormone (151 and 152 amino acids for man and rats, respectively). Human, porcine and bovine carboxy-terminal 28-amino acid peptides are identical and differ from similar peptides in rats and mice in that the former contain a methionine group at position 12 while the latter contain isoleucine. Various synthetic analogs of naturally occurring ANFs also have been found to have comparable biological activity. Examples of ANFs contemplated for use in this invention are α human AP 21 (atriopeptin I), human AP 28, α human AP 23 (atriopeptin II or APII), α human AP 24, α human AP 25, α human AP 26, α human AP 33, and the corresponding rat sequence of each of the above wherein Met 12 is Ile. See Table 1 for a comparison of the peptides.

TABLE 1

HUMAN PEPTIDE

AP 33 .... Leu Ala Gly Pro Arg Ser Leu Arg Arg Ser Ser Cys-S Phe Gly Gly Arg Met* Asp Arg Ile Gly Ala Gln Ser Gly Leu Gly Cys-S Asn Ser Phe Arg Tyr

AP 28 .......... Ser —— Tyr

AP 26 ........ Arg —— Tyr

AP 25 ........ Arg —— Tyr

AP 24 ........ Ser —— Tyr

AP 23 ........ Ser —— Arg

AP 21 ........ Ser —— Ser

* Ile in the rat peptide

A third aspect of the invention in the administration of a combination of an ACE inhibitor and a compound of formula I.

Examples of ACE inhibitors are those disclosed in the article by Wyvratt et al., cited above, and in the following U.S. patents: U.S. Patents 4,105,776, 4,468,519, 4,555,506, 4,374,829, 4,462,943, 4,470,973, 4,470,972, 4,350,704, 4,256,761, 4,344,949, 4,508,729, 4,512,924, 4,410,520 and 4,374,847, all incorporated herein by reference; and the following foreign patents or published patent applications:

British Specification No. 2095682 published October 6, 1982 discloses N-substituted-N-carboxyalkyl aminocarbonyl alkyl glycine derivatives which are said to be angiotensin converting enzyme inhibitors and have the formula

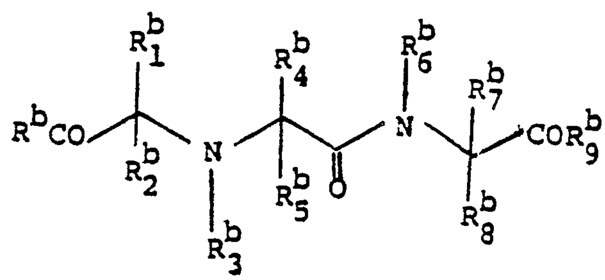

either

(A) $R^b$ and $R_9{}^b$ are OH, 1-6C alkoxy, 2-6C alkenyloxy, di-(1-6C alkyl)amino-(1-6C) alkoxy, 1-6C hydroxyalkoxy, acylamino-(1-6C)alkoxy, acyloxy-(1-6C)alkoxy, aryloxy, aryloxy-(1-6C)alkoxy, $NH_2$, mono- or di-(1-6C alkyl)amino, hydroxyamino or aryl-(1-6C)alkylamino;

$R_1{}^b$-$R_5{}^b$, $R_7{}^b$ and $R_8{}^b$ are 1-20C alkyl, 2-20C alkenyl, 2-20C alkynyl, aryl, aryl-(1-6C) alkyl having 7-12C or heterocyclyl-(1-6C)alkyl having 7-12C;

$R_6{}^b$ is cycloalkyl, polycycloalkyl, partly saturated cycloalkyl or polycycloalkyl, cycloalkyl-(1-6C)alkyl having 3-20C, 6-10C aryl, aryl-(1-6C)alkyl, aryl-(2-6C)alkenyl or aryl-(2-6C) alkynyl; or

$R_2{}^b$ and $R_3{}^b$ together with the C and N atoms to which they are attached or $R_3{}^b$ and $R_5{}^b$ together with the N and C atoms to which they are attached form an N-heterocycle containing 3-5C or 2-4C and a S atom;

all alkyl, alkenyl and alkynyl are optionally substituted by OH, 1-6C alkoxy, thio(sic), 1-6C alkylthio, $NH_2$, mono- or di(1-6C alkyl)amino, halogen or $NO_2$;

all 'cycloalkyl' groups (including poly and partially unsaturated) are optionally substituted by halogen, 1-6C

hydroxyalkyl, 1-6C alkoxy, amino-(1-6C alkyl)amino, di-(1-6C alkyl)amino, SH, 1-6C alkylthio, $NO_2$ or $CF_3$; and

aryl groups are optionally substituted by OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino; or

(B) $R^b$ and $R_9{}^b$ are H or 1-6C alkoxy;

$R_1{}^b$ and $R_2{}^b$ are H, 1-6C alkyl, aryl-(1-6C) alkyl having 7-12C or heterocyclyl-(1-6C) alkyl having 6-12C;

$R_3{}^b$-$R_5{}^b$, $R_7{}^b$ and $R_8{}^b$ are H or 1-6C alkyl;

$R_6{}^b$ is cycloalkyl, polycycloalkyl, partly saturated cycloalkyl or polycycloalkyl, cycloalkyl-(1-6C) alkyl having 3-20C, aryl or aryl-(1-6C) alkyl; and

aryl has 6-10C and is optionally substituted by 1-6C alkyl, 2-6C alkenyl, 2-6C alkynyl, OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino;

European Patent Application 0 050 800 published May 5, 1982 discloses carboxyalkyl dipeptides derivatives which are said to be angiotensin converting enzyme inhibitors and have the formula

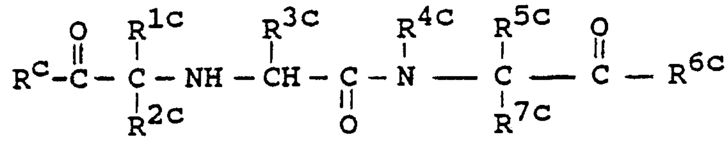

or a pharmaceutically acceptable salt thereof, wherein $R^c$ and $R^{6c}$ are the same or different and are hydroxy, lower alkoxy, lower alkenyloxy, dilower alkylamino lower alkoxy, acylamino lower alkoxy, acyloxy lower alkoxy, aryloxy, aryllower alkoxy, amino, lower alkylamino, dilower alkylamino, hydroxyamino, aryllower alkylamino, or substituted aryloxy or substituted aryllower alkoxy wherein the substitutent is methyl, halo or methoxy; $R^{1c}$ is hydrogen, alkyl of from 1 to 10 carbon atoms, substituted lower alkyl wherein the substitutent is hydroxy, lower alkoxy, aryloxy, substituted aryloxy, heteroaryloxy, substituted heteroaryloxy, amino, lower alkylamino, diloweralkylamino, acylamino, arylamino, substituted arylamino, guanidino, imidazolyl, indolyl, lower alkylthio, arylthio, substituted arylthio, carboxy, carbamoyl, lower alkoxy carbonyl, aryl, substituted aryl, aralkyloxy, substituted aralkyloxy, aralkylthio or substituted aralkylthio, wherein the aryl or heteroaryl portion of said substituted aryloxy, heteroaryloxy, arylamino, arylthio, aryl, aralkyloxy, aralkylthio group is substituted with a group selected from halo, lower alkyl, hydroxy, lower alkoxy, amino, aminomethyl, carboxyl, cyano, or sulfamoyl; $R^{2c}$ and $R^{7c}$ are the same or different and are hydrogen or lower alkyl; $R^{3c}$ is hydrogen, lower alkyl, phenyl lower alkyl, aminoethylphenyl lower alkyl, hydroxyphenyl lower alkyl, hydroxy lower alkyl, acylamino lower alkyl, amino lower alkyl, dimethylamino lower alkyl, guanidino lower alkyl, imidazolyl lower alkyl, indolyl lower alkyl, or lower alkyl thio lower alkyl; $R^{4c}$ and $R^{5c}$ are the same or different and are hydrogen, lower alkyl or $Z^c$, or $R^{4c}$ and $R^{5c}$ taken together form a group represented by $Q^c$, $U^c$, $V^c$, $Y^c$, $D^c$ or $E^c$, wherein;

$Z^c$ is

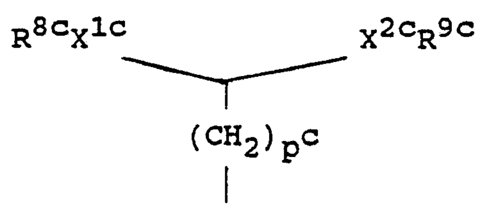

wherein $X^{1c}$ and $X^{2c}$ independent of each other are O, S or $CH_2$, $R^{8c}$ and $R^{9c}$ independent of each other are lower alkyl, lower alkenyl, lower alkynyl, cycloalkyl having 3 to 8 carbon atoms, hydroxy lower alkyl, or --$(CH_2)_n{}^cAr^c$, wherein $n^c$ is 0, 1, 2 or 3 and $Ar^c$ is unsubstituted or substituted phenyl, furyl, thienyl or pyridyl, wherein said substituted phenyl, furyl, thienyl or pyridyl groups are substituted with at least one group that is independently selected from $C_1$ to $C_4$ alkyl, lower alkoxy, lower alkylthio, halo, $CF_3$ and hydroxy, or $R^{8c}$ and $R^{9c}$ taken together form a bridge $W^c$, wherein $W^c$ is a single bond or a methylene bridge or a substituted methylene bridge when at least one of $X^{1c}$ and $X^{2c}$ is methylene, or $W^c$ is an alkylene or substituted alkylene bridge having 2 or 3 carbon atoms, said substituted methylene bridge or said

substituted alkylene bridge having one or two substituents selected from lower alkyl, aryl and aryl lower alkyl groups, and $p^c$ is 0, 1 or 2; with the proviso that at least one of $R^{4c}$ and $R^{5c}$ is $Z^c$, with the proviso that if $R^{4c}$ is $Z^c$ and $p^c$ is 0 then $X^{1c}$ and $X^{2c}$ must both be methylene, and with the proviso that if $X^{1c}$ and $X^{2c}$ are both methylene then $R^{8c}$ and $R^{9c}$ must form an alkylene bridge $W^c$;
$Q^c$ is

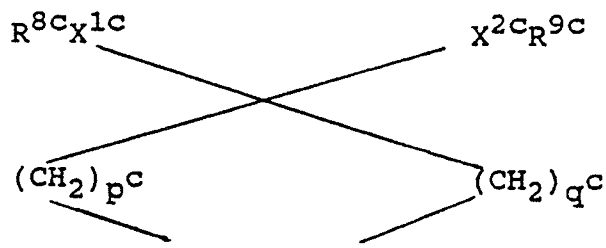

wherein $R^{8c}$, $R^{9c}$, $X^{1c}$ and $X^{2c}$ are as defined above, $p^c$ is 0, 1 or 2, $q^c$ is 0, 1 or 2, with the proviso that the sum of $p^c$ and $q^c$ must be 1, 2 or 3, with the proviso that if $p^c$ is 0 then $X^{1c}$ and $X^{2c}$ must be methylene, and with the proviso that if $X^{1c}$ and $X^{2c}$ are methylene then $R^{8c}$ and $R^{9c}$ taken together form a bridge $W^c$, wherein $W^c$ is as defined above;
$V^c$ is

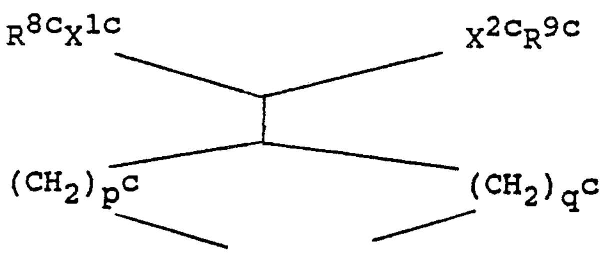

wherein $R^{8c}$, $R^{9c}$, $X^{1c}$ and $X^{2c}$ are as defined above, $p^c$ is 0, 1 or 2 and $q^c$ is 0, 1 or 2, with the proviso that the sum of $p^c$ and $q^c$ is 1, 2 or 3, with the proviso that if $X^{1c}$ and $X^{2c}$ are $CH_2$ then $R^{8c}$ and $R^{9c}$ taken together form a bridge $W^c$, wherein $W^c$ is as defined above;
$U^c$ is

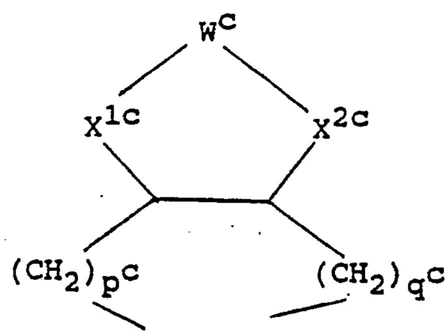

wherein $W^c$ is as defined above (except that $W^c$ may also be a methylene bridge when $X^{1c}$ and $X^{2c}$ are oxygen or sulfur), $X^{1c}$ and $X^{2c}$ are as defined above, $p^c$ is 0, 1 or 2, $q^c$ is 0, 1 or 2, with the proviso that the sum of $p^c$ and $q^c$ is 1 or 2, and with the proviso that if $p^c$ is 0, $X^{1c}$ must be $CH_2$;
$Y^c$ is

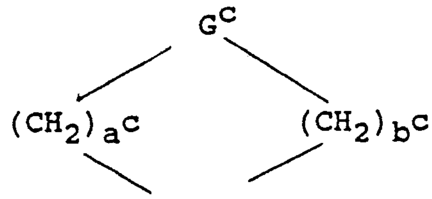

wherein $G^c$ is oxygen, sulfur or $CH_2$, $a^c$ is 2, 3 or 4 and $b^c$ is 1, 2, 3, 4 or 5, with the proviso that the sum of $a^c$ and $b^c$ is 5, 6 or 7 or $G^c$ is $CH_2$, $a^c$ is 0, 1, 2 or 3, $b^c$ is 0, 1, 2 or 3 with the proviso that the sum of $a^c$ and $b^c$ is 1, 2 or 3, with the proviso that the sum of $a^c$ and $b^c$ may be 1, 2 or 3 only if $R^{1c}$ is lower alkyl substituted with aralkylthio or aralkyloxy;

$D^c$ is

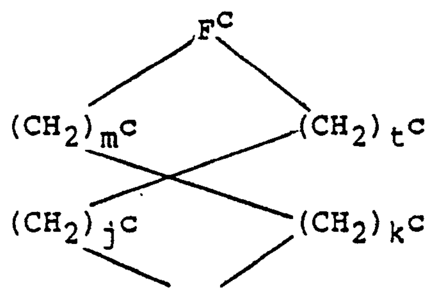

wherein $F^c$ is O or S, $j^c$ is 0, 1 or 2 and $k^c$ is 0, 1 or 2, with the proviso that the sum of $j^c$ and $k^c$ must be 1, 2 or 3, and $m^c$ is 1, 2 or 3 and $t^c$ is 1, 2 or 3, with the proviso that the sum of $m^c$ and $t^c$ must be 2, 3 or 4;

$E^c$ is

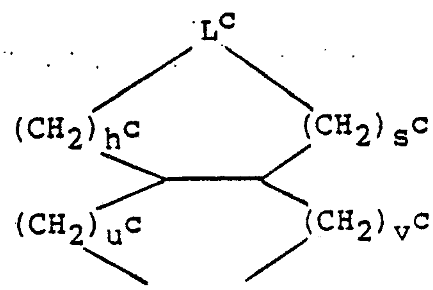

wherein $L^c$ is O or S, $u^c$ is 0, 1 or 2 and $v^c$ is 0, 1 or 2, with the proviso that the sum of $u^c$ and $v^c$ must be 1 or 2, and $h^c$ is 1 or 2 and $s^c$ is 1 or 2, with the proviso that the sum of $h^c$ and $s^c$ must be 2 or 3;

European Patent Application 0 079 522 published May 25, 1983 discloses N-carboxymethyl(amidino) lysylproline compounds which are said to be angiotensin converting enzyme inhibitors and have the formula where

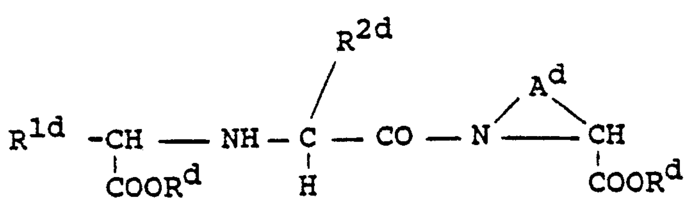

$(I)^d$

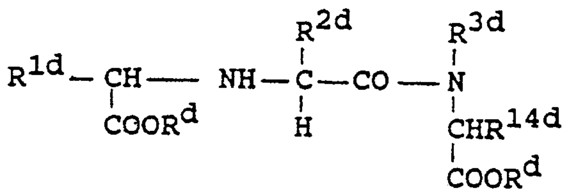

(Ia)$^d$

wherein:

$R^d$ and $R^{2d}$ are independently hydrogen;

loweralkyl; aralkyl; or aryl;

$R^{1d}$ is hydrogen; branched or straight chain $C_{1-12}$ alkyl and alkenyl; $C_3$-$C_9$ cycloalkyl and benzofused alkyl; substituted loweralkyl where the substituents are halo, hydroxy loweralkoxy, aryloxy, amino, mono- or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; arloweralkyl; arloweralkenyl; heteroarloweralkyl; heteroarloweralkenyl; substituted arloweralkyl, substituted arloweralkenyl, substituted heteroarloweralkyl, or substituted heteroarloweralkenyl where the aryl and heteroaryl substituents are halo, dihalo, loweralkyl, hydroxy, loweralkoxy, amino, aminoloweralkyl, acylamino, mono- or diloweralkylamino, carboxyl, haloloweralkyl, nitro, cyano, or sulfonamido, and where the loweralkyl portion of arloweralkyl may be substituted by amino, acylamino, or hydroxyl;

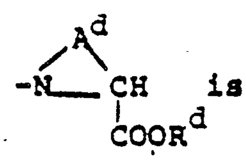 is 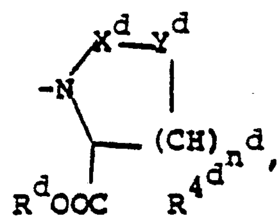 ,

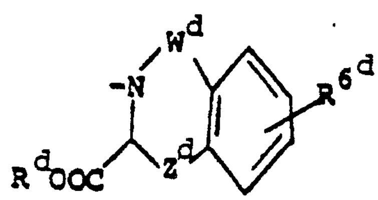

or 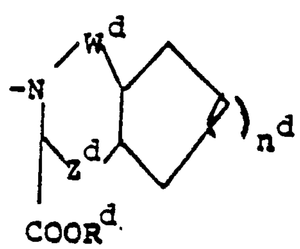

where:

$X^d$ and $Y^d$ taken together are $-CH_2$-$CH_2$-;

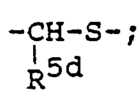 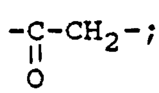 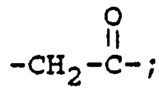 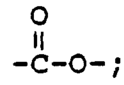 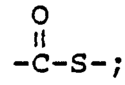

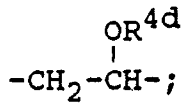 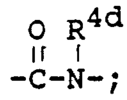 or 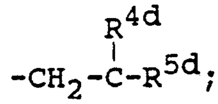

$R^{4d}$ is hydrogen; loweralkyl; aryl; substituted aryl;
$R^{5d}$ is hydrogen; loweralkyl; aryl or substituted aryl;
$n^d$ is 1 to 3;
$W^d$ is absent; $-CH_2-$; or

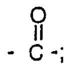

$Z^d$ is $-(CH_2)_m{}^d$, where $m^d$ is 0 to 2, provided that $m^d$ may not be 0 and $W^d$ may not be absent at the same time; and
$R^{6d}$ is hydrogen; loweralkyl; halo; or $OR^{4d}$;
$R^{2d}$ is $(CH_2)_r{}^d-B^d-(CH_2)_s{}^d-NR^{7d}R^{15d}$
where
$r^d$ and $s^d$ are independently 0 to 3;
$B^d$ is absent; -O-; -S-; or $-NR^{8d}$;
where $R^{8d}$ is hydrogen; loweralkyl; alkanoyl; or aroyl; and
$R^{7d}$ is

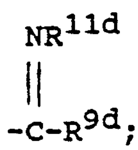 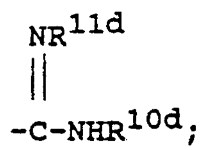 or 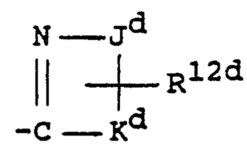

where
$R^{9d}$ is loweralkyl; aralkyl; aryl; heteroaryl; or heteroarloweralkyl and these groups substituted by hydroxy, lower alkoxy or halo; carboxyl; carboxamido; nitromethenyl.
$R^{10d}$ is hydrogen; loweralkyl; aryl; or amidino;
$R^{11d}$ hydrogen; loweralkyl; cyano; amidino; aryl; aroyl; loweralkanoyl;

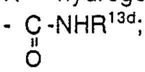

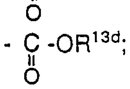

$-NO_2$; $-SO_2NH_2$; or $SO_2R^{13d}$;
$R^{12d}$ is hydrogen; loweralkyl; halo; aralkyl; amino; cyano; mono- or diloweralkylamino; or $OR^{4d}$;
$R^{13d}$ is hydrogen; loweralkyl; or aryl;
$R^{15d}$ is hydrogen; lower alkyl; aralkyl; or aryl;

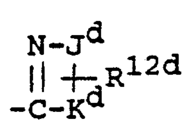

constitute a basic heterocycle of 5 or 6 atoms or benzofused analogs thereof and optionally containing 1-3 N atoms, an oxygen, a sulfur, an S=O, or an $SO_2$ group optionally substituted by amino, lower alkyl amino, diloweralkyl amino, lower alkoxy, or aralkyl groups;
$R^{3d}$ is $C_{3-8}$ cycloalkyl and benzofused $C_{3-8}$ cycloalkyl; perhydrobenzofused $C_{3-8}$ cycloalkyl; aryl; substituted aryl; heteraryl; substituted heteroaryl;
$R^{14d}$ is hydrogen or loweralkyl; and, a pharmaceutically acceptable salt thereof;

European Patent 79022 published May 18, 1983 discloses N-amino acyl-azabicyclooctane carboxylic

acid derivatives which have the formula

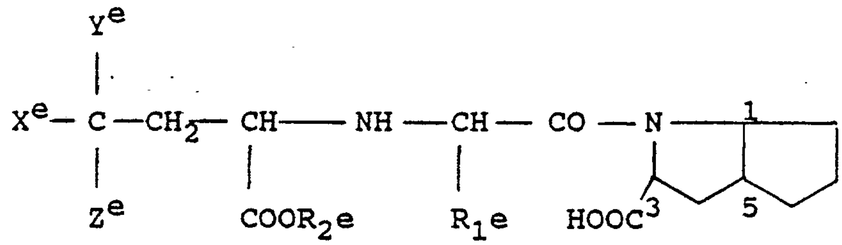

hydrogen atoms at ring positions 1 and 5 are cis to each other and the 3-carboxy group has the endo orientation;

$R_1{}^e$ is H, allyl, vinyl or the side chain of an optionally protected naturally occurring α-amino acid;

$R_2{}^e$ is H, 1-6C alkyl, 2-6C alkenyl or aryl(1-4C alkyl);

$Y^e$ is H or OH and $Z^e$ is H, or $Y^e$ and $Z^e$ together oxygen;

$X^e$ is 1-6C alkyl, 2-6C alkenyl, 5-9C cycloalkyl, 6-12C aryl (optionally substituted by one to three 1-4C alkyl or alkoxy, OH, halo, nitro, amino (optionally substituted by one or two 1-4C alkyl), or methylenedioxy) or indol-3-yl);

European Patent 46953 published March 10, 1982 discloses N-amino acyl-indoline and tetrahydro isoquinoline carboxylic acids which are angiotensin coverting enzyme inhibitors and have the formula

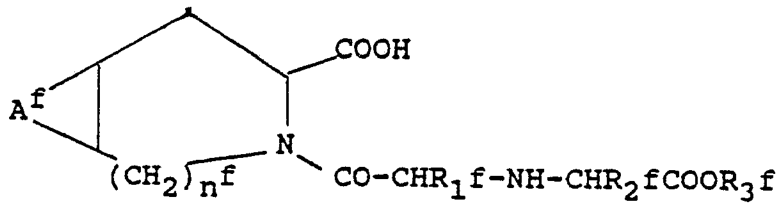

$n^f$ is 0 or 1;

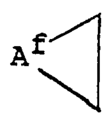

isa benzene or cyclohexane ring:

$R_1{}^f$ and $R_2{}^f$ are each 1-6C alkyl, 2-6C alkenyl, 5-7C cycloalkyl, 5-7C cycloalkenyl, 7-12C cycloalkylalkyl, optionally partially hydrogenated 6-10C aryl, 7-14C aralkyl or 5-7 membered monocyclic or 8-10 membered bicyclic heterocyclyl containing 1 or 2 S or O and/or 1-4N atoms; all $R_1{}^f$ and $R_2{}^f$ groups are optionally substituted,

$R_3{}^f$ is H, 1-6C alkyl, 2-6C alkenyl or 7-14C aralkyl.

The following Table II lists ACE inhibitors preferred for use in the combination of this invention.

## TABLE II

## PREFERRED ACE INHIBITORS

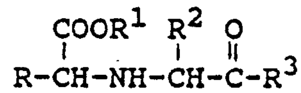

| | R | $R_1$ | $R_2$ | $R_3$ |
|---|---|---|---|---|
| spirapril | $C_6H_5CH_2CH_2$- | Et | $CH_3$ | 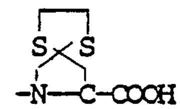 |
| enalapril | $C_6H_5CH_2CH_2$- | Et | $CH_3$ | prolyl |
| ramipril | $C_6H_5CH_2CH_2$- | Et | $CH_3$ | 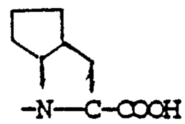 |
| perindopril | $CH_3CH_2CH_2$ | Et | $CH_3$ | 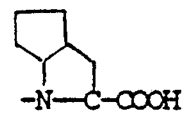 |
| indolapril | $C_6H_5CH_2CH_2$- | Et | $CH_3$ | 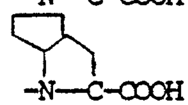 |
| lysinopril | $C_6H_5CH_2CH_2$- | H | $NH_2(CH_2)_4$- | prolyl |
| CI-925 | $C_6H_5CH_2CH_2$- | Et | $CH_3$ | 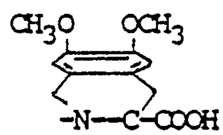 |
| pentopril (NH = $CH_2$) | $CH_3$ | Et | $CH_3$ | 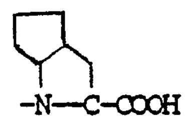 |
| cilazapril | $C_6H_5CH_2CH_2$- | H | 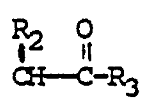 | 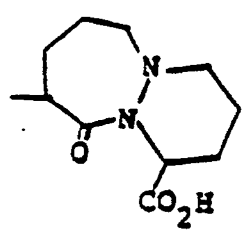 |

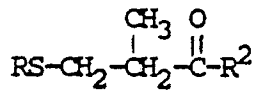

| | R | $R_2$ |
|---|---|---|
| captopril | H | prolyl |
| zofenopril | $C_6H_5CO-$ | 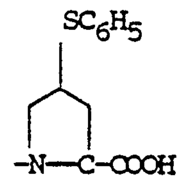 |
| pivalopril | 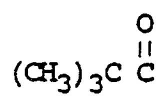 | 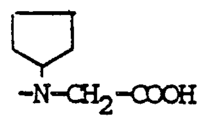 |

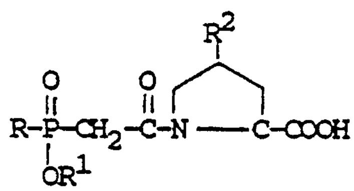

| | R | $R^1$ | $R^2$ |
|---|---|---|---|
| fosinopril | $C_6H_5-(CH_2)_4-$ | 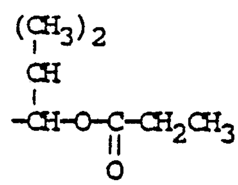 | $C_6H_5-$ |

We have found that the novel compounds of the present invention are effective in treating congestive heart failure and various types of hypertension, particularly volume expanded hypertension. These novel compounds as well as other mercapto-acylamino acids known in the art have been found to enhance both the magnitude and duration of the antihypertensive and natriuretic effects of endogenous ANF. Administration of a combination of a mercapto-acylamino acid and an ACE inhibitor provides an antihypertensive effect greater than either the mercapto-acylamino acid or ACE inhibitor alone. Administration of a combination of a mercapto-acylamino acid of formula I and an exogenous ANF or ACE inhibitor is therefore particularly useful in treating hypertension.

In addition to the compound aspect, the present invention therefore also relates to treating hypertension with a mercapto-acylamino acid of formula I or with a mercapto-acylamino acid of formula I in combination with an ANF or an ACE inhibitor, which methods comprise administering to a mammal in need of such treatment an antihypertensive effective amount of the mercapto-acylamino acid or an antihypertensive effective amount of a combination of a mercapto-acylamino acid and ANF or ACE inhibitor. The drug or combination of drugs is preferably administered in a pharmaceutically acceptable carrier, e.g. for oral or parenteral administration. The combinations of drugs may be co-administered in a single composition, or

components of the combination therapy may be administered separately. Where the components are administered separately, any convenient combination of dosage forms may be used, e.g. oral mercapto-acylamino acid/oral ANF, oral mercapto-acylamino acid/parenteral ACE inhibitor, parenteral mercapto-acylamino acid/oral ANF, parenteral mercapto-acylamino acid/parenteral ACE inhibitor.

When the components of a combination of a mercapto-acylamino acid and an ANF are administered separately, it is preferred that the mercapto-acylamino acid be administered first.

The present invention also relates to a pharmaceutical composition comprising a mercapto-acylamino acid for use in treating hypertension, to a pharmaceutical composition comprising both a mercapto-acylamino acid and an ANF and to a pharmaceutical composition comprising both a mercapto-acylamino acid and an ACE inhibitor.

The antihypertensive effect of mercapto-acylamino acids alone and in combination with ACE inhibitors was determined according to the following procedure:

Male Sprague Dawley rats weighing 100-150 g were anesthetized with ether and the right kidney was removed. Three pellets containing Doc acetate (desoxycorticosterone acetate, DOCA, 25 mg/pellet) were implanted subcutaneously. Animals recovered from surgery, were maintained on normal rat chow and were allowed free access to a fluid of 1% NaCl and 0.2% KCl instead of tap water for a period of 17-30 days. This procedure results in a sustained elevation in blood pressure and is a slight modification of published procedures (e.g. Brock et al., 1982) that have been used to produce DOCA salt hypertension in the rat.

On the day of study, animals were again anesthetized with ether and the caudal artery was cannulated for blood pressure measurement. Patency of the caudal artery cannula was maintained with a continuous infusion of dextrose in water at a rate of 0.2 ml/hr. Animals were placed into restraining cages where they recovered consciousness. Blood pressure was measured from caudal artery catheter using a Statham pressure transducer attached to a Beckman oscillographic recorder. In addition, a cardiovascular monitoring device (Buxco Electronics, Inc.) and a digital computer were used to calculate average blood pressures.

After an equilibration period of at least 1.5 hr., animals were dosed subcutaneously (1 ml/kg) with vehicle (methylcellulose, hereinafter MC) or mercapto-acylamino acid and blood pressure was monitored for the next 4 hours.

The antihypertensive effect of mercapto-acylamino acids in combination with ANF was determined according to the following procedures:

Male spontaneously hypertensive rats (SHR), 16-18 weeks old, 270-350 g, were anesthetized with ether and the abdominal aorta was cannulated through the tail artery. The animals were then placed into restrainers to recover from anesthesia (in less than 10 min.) and remained inside throughout the experiments. Through a pressure transducer (Gould P23 series) analog blood pressure signals were registered on a Beckman 612 recorder. A Buxco digital computer was used to obtain mean arterial pressures. Patency of the arterial cannula was maintained with a continuous infusion of 5% dextrose at 0.2 ml/hr. Animals were allowed a 90-min equilibration period. The animals first underwent a challenge with an ANF such as atriopeptin II (AP II) or AP28 30 µg/kg iv and at the end of 60 min. were treated with drug vehicle or a mercapto-acylamino acid subcutaneously. A second ANF challenge was administered 15 min. later and blood pressure was monitored for the next 90 min.

The antihypertensive effect in SHR of mercapto-acylamino acids and ACE inhibitors, alone and in combination, can be determined as follows:

Animals are prepared for blood pressure measurement as described above. After stabilization, animals are dosed subcutaneously or orally with test drugs or placebo and blood pressure is monitored for the next 4 hr.

The compositions of this invention comprise a mercapto-acylamino acid or a mercapto-acylamino acid and an ANF or a mercapto-acylamino acid and an ACE inhibitor in combination with a pharmaceutically acceptable carrier for administration to mammals. A variety of pharmaceutical forms is suitable, preferably for oral or parenteral administration, although mechanical delivery systems such as transdermal dosage forms are also contemplated.

The daily antihypertensive dose of the compound or combinations of this invention is as follows: for mercapto-acylamino acids alone the typical dosage is 1 to 100 mg/kg of mammalian weight per day administered in single or divided dosages; for the combination of mercapto-acylamino acid and an ANF, the typical dosage is 1 to 100 mg of mercapto-acylamino acid/kg mammalian weight per day in single or divided dosages plus 0.001 to 0.1 mg ANF/kg of mammalian weight per day, in single or divided dosages, and for the combination of mercapto-acylamino acid and an ACE inhibitor, the typical dosage is 1 to 100 mg of mercapto-acylamino acid/kg mammalian weight per day in single or divided dosages plus 0.1 to 30 mg ACE inhibitor/kg of mammalian weight per day in single or divided dosages. The exact dose of any component or combination to be administered is determined by the attending clinician and is dependent on

the potency of the compound administered, the age, weight, condition and response of the patient.

Generally, in treating humans having hypertension, the compounds or combinations of this invention may be administered to patients in a dosage range as follows: for treatment with mercapto-acylamino acids alone, about 10 to about 500 mg per dose given 1 to 4 times a day, giving a total daily dose of about 10 to 2000 mg per day; for the combination of mercapto-acylamino acid and ANF, about 10 to about 500 mg mercapto-acylamino acid per dose given 1 to 4 times a day and about 0.001 to about 1 mg ANF given 1 to 6 times a day (total daily dosage range of 10 to 2000 mg day and .001 to 6 mg/day, respectively); and for the combination of a mercapto-acylamino acid and an ACE inhibitor, about 10 to about 500 mg mercapto acylamino acid per dose given 1 to 4 times a day and about 5 to about 50 mg ACE inhibitor given 1 to 3 times a day (total daily dosage range of 10 to 2000 mg/day and 5 to 150 mg/day, respectively). Where the components of a combination are administered separately, the number of doses of each component given per day may not necessarily be the same, e.g. where one component may have a greater duration of activity, and will therefore need to be administered less frequently.

Typical oral formulations include tablets, capsules, syrups, elixirs and suspensions. Typical injectable formulations include solutions and suspensions.

The typical acceptable pharmaceutical carriers for use in the formulations described above are exemplified by: sugars such as lactose, sucrose, mannitol and sorbitol, starches such as cornstarch, tapioca starch and potato starch; ceullulose and derivatives such as sodium carboxymethyl cellulose, ethyl cellulose and methyl cellulose; calcium phosphates such as dicalcium phosphate and tricalcium phosphate; sodium sulfate; calcium sufate; polyvinylpyrrolidone, polyvinyl alcohol; stearic acid; alkaline earth metal stearates such as magnesium stearate and calcium stearate, stearic acid, vegetable oils such as peanut oil, cottonseed oil, sesame oil, olive oil and corn oil; non-ionic, cationic and anionic surfactants; ethylene gylcol polymers; beta-cyclodextrin; fatty alcohols and hydrolyzed cereal solids; as well as other nontoxic compatible fillers, binders, disintegrants, buffers, preservatives, anti-oxidants, lubricants, flavoring agents, and the like commonly used in pharmaceutical formulations.

Since the present invention relates to treatment of hypertension with a combination of active ingredients wherein said active ingredients may be administered separately, the invention also relates to combining separate pharmaceutical compositions in kit form. That is, two kits are contemplated, each combining two separate units: a mercapto-acylamino acid pharmaceutical composition and an ANF pharmaceutical composition in one kit and a mercapto-acylamino acid pharmaceutical composition and an ACE inhibitor pharmaceutical composition in a second kit. The kit form is particularly advantageous when the separate components must be administered in different dosage forms (e.g. oral and parenteral) or are administered at different dosage intervals.

Following are examples of procedures for preparing compounds of formula I.

## EXAMPLE 1

### S-ACETYL-N-(3-PHENYL-2-(MERCAPTOMETHYL)PROPIONYL)-(S)-4-METHYLMERCAPTO)-METHIONINE METHYL ESTER

A. N-Carbobenzyloxy-(S)-4-(Methylmercapto)methionine Methyl Ester

Dissolve N-carbobenzyloxy-(S)-allylglycine methyl ester (3.8g) in methylene chloride ($CH_2Cl_2$) (70 mL), cool to -78 °C, and treat with ozone until the solution turns blue. Purge the system with nitrogen, add dimethylsulfide (15 mL), and allow the reaction to warm to 0 °C. Treat with gaseous methyl mercaptan followed by a catalytic amount of gaseous hydrogen chloride (HCl). Stir 1 hour, evaporate the solvent, and dissolve the resultant residue in ethyl acetate (EtOAC). Wash with saturated aqueous sodium bicarbonate ($NaHCO_3$) and sodium NaCl), dry the organic layer over magnesium sulfate ($MgSO_4$), filter, and evaporate the solvent to obtain the title compound. $R_f$ (silica gel; hexane:EtOAc, 70:30) = 0.52.

B. (S)-4-(Methylmercapto)methionine Methyl Ester

Dissolve the product prepared in Part A (2.4g) in trifluoroacetic acid (TFA) (152 mL) and thioanisole (43 mL) and stir 12 hours. Evaporate the solvent and partition the resultant residue between 1N HCl (aq.) (125 mL) and EtOAC (125 mL). Cool the aqueous layer to 0° C, adjust the pH to 9 with concentrated ammonium hydroxide, and extract the desired product into EtOAC. Dry the organic layer over sodium sulfate ($Na_2SO_4$), filter, and evaporate the solvent to obtain the title compound. $R^f$ (silica gel; $CH_2Cl_2$:$CH_3OH$:$NH_4OH$, 989:10:1) = 0.49.

C. S-Acetyl-N-(3-Phenyl-2-(Mercaptomethyl)propionyl)-(S)-4-(Methylmercapto)methionine Methyl Ester

Dissolve the product of Part B (0.86g), S-acetyl-3-phenyl-2-(mercaptomethyl)propionic acid (1.22g), and HOBT (0.69g) in $CH_2Cl_2$ (10 mL). Add CMC (0.98g), stir 12 hours, and evaporate the solvent. Dissolve the resultant residue in diethyl ether ($Et_2O$), wash with 5% citric acid (aq.), saturated $NaHCO_3$ (aq.), and saturated NaCl, and dry over $MgSO_4$. Filter and evaporate the solvent to give a mixture of diastereomers of the title compound. $R_f$ (silica gel; hexane:EtOAc, 80:20) = 0.19 and 0.24. Separate the diastereomers on normal phase HPLC (silica gel; hexane:EtOAc, 80:20). Evaporate the solvent from the respective fractions to obtain the resolved title compounds, (i.e. the S,S and R,S diastereomers); FAB mass spec m/e = 430 (M + H).

## EXAMPLE 2

N-(3-PHENYL-2-(MERCAPTOMETHYL)PROPIONYL)-(S)-4-(METHYLMERCAPTO)METHIONINE AMIDE

Dissolve a pure diastereomer prepared in Part C of Example 1 (0.2g) in $CH_3OH$ (30 mL) and bubble gaseous ammonia ($NH_3$) through the solution until saturated. Seal the reaction vessel, stir 15 hours, and evaporate the solvent. Triturate the resultant residue in $Et_2O$ and filter the solid. $R_f$ (silica gel; $CH_2Cl_2$:$CH_3OH$:$CH_3COOH$, 969:30:1) = 0.38. Purify on reverse phase HPLC (C-18 column; $H_2O$:$CH_3CN$:$CF_3COOH$, 449:549:2). Collect the desired fractions and evaporate the solvent to give the title compound. FAB mass spec m/w = 373 (M + H).

## EXAMPLE 3

N-(3-PHENYL-2-(MERCAPTOMETHYL)PROPIONYL)-(S)-4-(METHYLMERCAPTO)METHIONINE

Dissolve a pure diastereomer prepared in Part C of Example 1 (.22g) in $CH_3OH$ (1 mL) and treat with 1N sodium hydroxide (aq.) (1.43 mL). Stir 2 hours and acidify with 1N HCl (aq) (3 mL). Extract the product into EtOAC, dry the organic layer over $Na_2SO_4$, filter, and evaporate the solvent. $R_f$ (silica gel; $CH_2Cl_2$:$CH_3OH$:$CH_3COOH$, 969:30:1) = 0.60. Purify on reverse phase HPLC (C-18 column; $H_2O$:$CH_3CN$:$CF_3COOH$, 549:449:2). Collect the desired fractions and evaporate the solvent to obtain the title compound. FAB mass spec m/e = 374 (M + H).

## EXAMPLE 4

S-PIVALOYLOXY-N-(3-PHENYL-2-(MERCAPTOMETHYL)PROPIONYL)-(S)-4-(METHYLMERCAPTO)-METHIONINE

Add chloromethyl pivalate (.32 ml) to a stirred suspension of the product of Example 3 (.74g) and anhydrous potassium carbonate (.58g) in anhydrous DMF (15 ml). Stir 12 hours at room temperature, filter and evaporate the filtrate in vacuo. Add EtOAC and 1N HCl. Dry the organic layer over $MgSO_4$, filter and evaporate the solvent to obtain the title compound.

## EXAMPLE 5

S-ACETYL-N-(3-PHENYL-2-(MERCAPTOMETHYL)PROPIONYL)-(S)-4-(METHYLMERCAPTO)METHIONINE,2-[N,N-DI-(2-HYDROXYETHYL)GLYCOLAMIDE ESTER]

A. N-Carbobenzyloxy-(S)-4-(Methylmercapto)-methionine

Dissolve the product of Example 1, Part A (23.3g) in MeOH (75 ml) and add dropwise over 0.5 hours 1N NaOH (aq.) (71.12 ml). Stir 30 minutes, concentrate to 50 ml, acidify to pH 3.0 with concentrated HCl, and extract the product with EtOAC. Dry the organic layer over $MgSO_4$ with activated carbon, filter and evaporate the solvent to obtain the title compound. $R_f$ (silica gel; $CH_2Cl_2$:$CH_3OH$:AcOH, 979:20:1) = 0.48.

B. (S)-4-(Methylmercapto)methionine

Dissolve the product of Part A (19.74g) in TFA (100 ml) and add thioanisole (10 ml). Stir 3 hours, dilute with water (250 ml) and ether (100 ml), and evaporate the aqueous layer to obtain the title compound.

C. S-Acetyl-N-(3-Phenyl-2-(Mercaptomethyl)propionyl)(S)-4-(Methylmercapto)methionine

To the product of Part B (12.3g) and triethylamine (8.78 ml) in $CH_3CN$:$H_2O$ (2:1, 300 ml), add S-Acetyl-3-Phenyl-2-(mercaptomethyl)propionyl chloride in $CH_3CN$ (70 ml) dropwise. Stir 1 hour, acidify with 1N HCl (100 ml) and extract with $Et_2O$ (600 ml). Dry the organic layer over $MgSO_4$, filter and evaporate the solvent to obtain a mixture of diastereomers of the title compound. $R_f$ (silica gel; $CH_2Cl$:MeOH:AcOH, 98.5:1.0:0.5) = 0.20, 0.18. Purify the products on a flash chromatographic column.

D. S-Acetyl-N-(3-Phenyl-2-(Mercaptomethyl)propionyl)-(S)-4-(Methylmercapto)methionine, 2-glycolic acid ester

To the product of Part C (1.3g) dissolved in $H_2O$:$CH_3OH$ (1:1, 25 ml), add cesium carbonate (.508g) in $H_2O$ (2 ml), and evaporate the solvent. Dissolve the residue in DMF (8 ml) and add t-butyl bromoacetate. Stir 4 hours, evaporate the solvent, suspend the resultant mixture in EtOAC, and wash with $H_2O$. Dry over $MgSO_4$, filter and evaporate the solvent. Dissolve the residue in $CH_2Cl_2$ (30 ml) and bubble HCl gas through the solution for 5 minutes. Stir 24 hours and evaporate the solvent to obtain the title compound. $R_f$ - (silica gel; $CH_2Cl_2$:MeOH:AcOH, 90:5:5) = 0.12.

E. S-Acetyl-N-(3-Phenyl-2-(Mercaptomethyl)propionyl)(S)-4-(Methylmercapto)methionine-2-[N,N-Di(2-hydroxyethyl)glycolamide ester]

To the product of Part D (.74g) in THF (4 ml), add N-methylmorpholine (.196 ml), cool to -15° C, add isobutylchloroformate (.231 ml), and diethanolamine (.256 ml). Stir 1 hour and evaporate the solvent. Purify on flash chromatography to obtain the title compound. $R_f$ (silica gel; $CH_2Cl_2$:MeOH, 96:4) - 0.39; FAB mass spec. m/e = 561 (M+H).

## EXAMPLE 6

### N-(3-PHENYL-2-(MERCAPTOMETHYL)PROPIONYL)-(S)-2-(1,3-DITHIANE)ALANINE

A. N-Carbobenzyloxy-(S)-2-(1,3-Dithiane)alanine Methyl Ester

Dissolve N-carbobenzyloxy-(S)-allylglycine methyl ester (6.5g) in $CH_2Cl_2$ (70 ml), cool to -78° C, and treat with ozone until the solution turns blue. Purge the system with nitrogen and add dimethylsulfide (25 ml). Add 1,3-propanedithiol (22.5 ml) followed by a catalytic amount of HCl gas. Stir 2 hours at -78° C and allow the mixture to warm to room temperature. Evaporate the solvent, dissolve the residue in EtOAC, and wash with saturated $NaHCO_3$ (aq) and saturated NaCl. Dry the organic layer over magnesium sulfate, filter, and evaporate the solvent to obtain the title compound. $R_f$ (silica gel; EtOAc:Hexane, 20:80) = 0.31.

B.

Treat the product of Part A in a manner similar to that described in Parts B and C of Example 1, followed by hydrolysis as described in Example 3 to obtain the title compound. FAB mass spec. m/e = 386 (M+H).

Using procedures similar to those described above and in the examples, prepare the following compounds of formula I wherein R is $-CH(SCH_3)_2$, $R^1$ hydrogen, $R_2$ is acetyl and n is 1:

| Example # | $R_3$ | diastereomer | FAB mass spec. m/e (M+1) |
|---|---|---|---|
| 7 | $-O(CH_2)_2OCH_3$ | S,S | 474 |
| 7a | " | R,S | 474 |
| 8 | $-OCH_2\overset{O}{\overset{\|}{C}}-N(CH_3)_2.$ | S,S | 501 |
| 8a | " | R,S | 501 |
| 9 | $-OCH_2\underset{\overset{\|}{O}}{\overset{CH_3}{\overset{\mid}{C}}}N(CH_2)_2N(CH_3)_2$ | S,S | 595 |
| 10 | $-NH_2$ | R,S | 415 |
| 10A | " | S,S | 415 |

Examples of formulations follow. "Active" refers to any mercapto-acylamino acid of formula I. "ACE inhibitor" refers to any of the ACE inhibitors listed earlier, especially those listed in the table of preferred

ACE inhibitors. "ANF" refers to any atrial natriuretic factor especially those listed in Table 1.

## EXAMPLE 11

Tablets

| Formula No. | Ingredient | mg/tablet | mg/tablet |
|---|---|---|---|
| 1 | Active | 50 | 500 |
| 2 | Lactose | 122 | 213 |
| 3 | Corn Starch, Food Grade, as a 10% paste in Purified Water | 30 | 40 |
| 4 | Corn Starch, Food Grade | 45 | 40 |
| 5 | Magnesium Stearate | 3 | 7 |
| | Approximate Table Weight | 250 | 700 |

Method of Manufacture

Mix Items Nos. 1 and 2 in a suitable mixer for 10-15 minutes. Granulate the mixture with Item No. 3. Mill the damp granules through a coarse screen (e.g., 1/4") if needed. Dry the damp granules. Screen the dried granules if needed and mix with Item No. 4 for 10-15 minutes. Add Item No. 5 and mix for 1-3 minutes. Compress the mixture to appropriate size and weight on a suitable tablet machine.

## EXAMPLE 12

Sterile powder for injection:

| | g/vial | g/vial |
|---|---|---|
| Active Sterile Powder | 0.5 | 1.0 |

Add sterile water for injection or bacteriostatic water for injection for reconstitution.

## EXAMPLE 13

Injectable Solution:

| Ingredient | mg/ml | mg/ml |
|---|---|---|
| Active | 100 | 100 |
| Methylparaben | 1.8 | 1.8 |
| Propylparaben | 0.2 | 0.2 |
| Sodium Bisulfite | 3.2 | 3.2 |
| Disodium Edetate | 0.1 | 0.1 |
| Sodium Sulfate | 2.6 | 2.6 |
| Water for Injection q.s. ad | 1.0 ml | 1.0 ml |

Method of Manufacture

Dissolve parabens in a portion (85% of the final volume) of the water for injection at 65-70°C. Cool to 25-35°C. Charge and dissolve the sodium bisulfite, disodium edetate and sodium sulfate. Charge and dissolve the active. Bring the solution to final volume by adding water for injection. Filter the solution through 0.22μ membrane and fill into appropriate containers. Terminally sterilize the units by autoclaving.

EXAMPLE 14

Tablets

| Formula No. | Ingredient | mg/tablet | mg/tablet |
|---|---|---|---|
| 1 | Active | 50 | 400 |
| 2 | ACE inhibitor | 25 | 50 |
| 3 | Lactose | 97 | 188 |
| 4 | Corn Starch, Food Grade, as a 10% Paste in Purified Water | 30 | 40 |
| 5 | Corn Starch, Food Grade | 45 | 40 |
| 6 | Magnesium Stearate | 3 | 7 |
| | Approximate Table Weight | 250 | 700 |

Method of Manufacturing

Prepare the tablets as in Example 1.

EXAMPLE 15

ACE inhibitor Tablets

Substitute an ACE inhibitor for the active in Example 13.

EXAMPLE 16

Tablets

| Formula No. | Ingredient | mg/tablet | mg/tablet |
|---|---|---|---|
| 1 | Active | 50 | 400 |
| 2 | ANF | 0.1 | 1 |
| 3 | Lactose | 121.9 | 212 |
| 4 | Corn Starch, Food Grade, as a 10% paste in Purified Water | 30 | 40 |
| 5 | Corn Starch, Food Grade | 45 | 40 |
| 6 | Magnesium Stearate | 3 | 7 |
| | | 250 | 700 |

Method of Manufacturing

Prepare the tablets as in Example 1.

EXAMPLE 17

Tablets

| Formula No. | Ingredient | mg/tablet | mg/tablet |
|---|---|---|---|
| 1 | ANF | 0.1 | 1 |
| 2 | Lactose | 61 | 121 |
| 3 | Corn Starch, Food Grade, as a 10% paste in Purified Water | 15 | 30 |
| 4 | Corn Starch, Food Grade | 22.4 | 45 |
| 5 | Magnesium Stearate | 1.5 | 3 |
| | | 100 | 200 |

Method of Manufacture

Prepare tablets as in Example 1.

**Claims**

1. A compound having the structural formula I

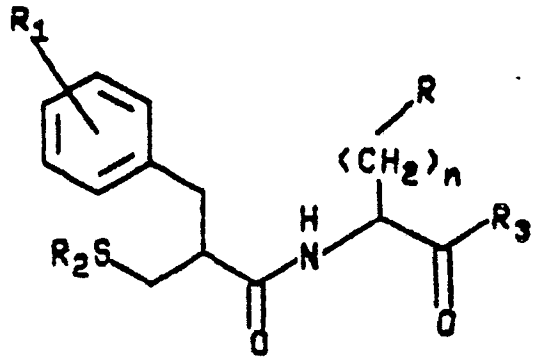

I

wherein n is 0-4;
R is

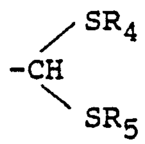 , 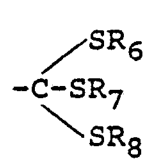 , 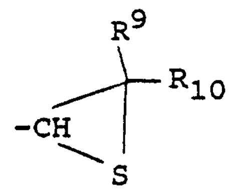 ;

$R_1$ is 1-3 substituents selected from the group consisting of hydrogen, halogeno, lower alkyl, cyclolower alkyl, lower alkoxy, hydroxy, aryl, aryloxy, cyano, aminomethyl, carboxy, lower alkoxy carbonyl and carbamyl;

$R_2$ is hydrogen,
$R_{11}-\overset{O}{\overset{\|}{C}}-$ or $R_{11}-\overset{O}{\overset{\|}{C}}-OCH_2-$, wherein $R_{11}$ is lower alkyl, aryl or arylmethyl;

$R^3$ is $-OR_{12}$,

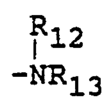 or 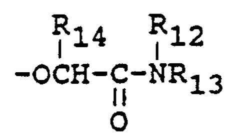 ;

$R_4$, $R_5$, $R_6$, $R_7$, $R_8$, $R_9$ and $R_{10}$ are independently lower alkyl, aryl lower alkyl, (cyclolower alkyl) lower alkyl, substituted aryl lower alkyl or substituted (cyclolower alkyl) lower alkyl, wherein in the substituents on the aryl and cyclolower alkyl portions are 1-3 substituents selected from the group consisting of lower alkyl, hydroxy, halogeno, lower alkoxy and amino, or $R_4$ and $R_5$ are alkyl and together with the sulfur and carbon atoms to which they are attached form a 5-7 membered ring;

$R_{12}$ and $R_{13}$ are independently hydrogen, lower alkyl or substituted lower alkyl wherein the substituents are selected from the group consisting of 1 or 2 hydroxy groups, 1 or 2 lower alkoxy groups, lower alkoxy lower alkoxy, halogeno, halogeno lower alkoxy, amino, mono- or di-lower alkylamino, aryl, substituted aryl wherein the substituents on aryl are 1-3 substituents selected from the group consisting of lower alkyl, hydroxy, halogeno, lower alkoxy and amino, and a 5-6 membered saturated ring comprising 1-2 oxygen atoms as ring members wherein the ring carbon atoms can be substituted with 0-2 lower alkyl substituents; or $R_{12}$ and $R_{13}$ together with the nitrogen to which they are attached complete a 5-7 membered ring, wherein one of the 4-6 ring members comprising $R_{12}$ and $R_{13}$ may be a nitrogen atom, an alkyl-substituted nitrogen atom or an oxygen atom, and wherein the ring may be substituted on the ring carbon atoms with substituents chosen from alkyl and hydroxy groups;

$R_{14}$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl or carbamoylalkyl;
and the pharmaceutically acceptable salts thereof.

2. A compound of claim 1 wherein R is

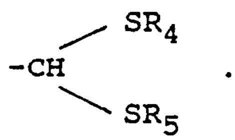

3. A compound of claim 1 wherein R is

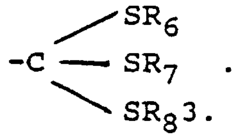

4. A compound of claim 1 wherein R is

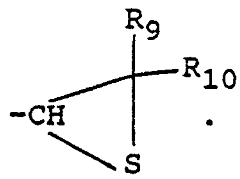

5. A compound of claim 1, 2, 3 or 4 wherein $R_1$ is hydrogen, methyl, methoxy or halogeno.

6. A compound of claim 1, 2, 3, 4, or 5 wherein $R_2$ is hydrogen, acetyl or benzoyl.

7. A compound of claim 1, 2, 3, 4, 5, or 6 wherein $R_3$ is hydroxy, alkoxy or amino.

8. A compound of claim 1, 2, 3, 4, 5, 6, or 7 wherein n is 1.

9. A compound of claim 1, 2, 5, 6, 7, or 8 wherein $R_4$ and $R_5$ are each lower alkyl.

10. A compound of claim 1, 3, 5, 6, 7 or 8 wherein $R_6$,$R_7$ and $R_8$ are each lower alkyl.

11. A compound of claim 1, 4, 5, 6, 7 or 8 wherein $R_9$ and $R_{10}$ are each hydrogen.

12. An (S,S) or (R,S) isomer of a compound of claim 1 named N-(3-phenyl-2-(mercaptomethyl)-propionyl)(S)-4-(methylmercapto)methionine.

13. A method for treating hypertension or congestive heart failure in mammals comprising administering to a mammal in need of such treatment an antihypertensive effective amount of a compound of claim 1, alone or in combination with an atrial natruiretic factor or an angiotenin converting enzyme inhibitor.

14. An antihypertensive pharmaceutical composition comprising an antihypertensive effective amount of a compound of claim 1-12 in a pharmaceutically acceptable carrier, alone or in combination with an atrial natriuretic factor or an angiotenin converting enzyme inhibitor.

15. A method of claim 13 wherein the atrial natriuretic peptide is chosen from human AP 21, $\alpha$ human AP 28, $\alpha$ human AP 23, $\alpha$ human AP 24, $\alpha$ human AP 25, $\alpha$ human AP 26, $\alpha$ human AP 33, and the corresponding atrial peptides wherein the methionine at position 12 is replaced by isoleucine.

16. A kit comprising in separate containers in a single package pharmaceutical compositions for use in combination to treat hypertension in mammals which comprises in one container a pharmaceutical composition comprising a mercapto-acylamino acid and in a second container a pharmaceutical composition comprising an atrial natriuretic factor.

17. A method of claim 13 wherein the angiotensin coverting enzyme inhibitor is selected from: spirapril, enalapril, ramipril, perindopril, indolapril, lysinopril, [3S-[2R,(R′),3R]]-2-[2-[[1-(ethoxycarbonyl)-3-phenylpropyl]amino]-1-oxopropyl]-1,2,3,4-tetrahydro-6,7-dimethoxy-3-isoquinoline-carboxylic acid, pentopril, cilazapril, captopril, zofenopril, pivalopril and fosinopril.

18. A kit comprising in separate containers in a single package pharmaceutical compositions for use in combination to treat hypertension in mammals which comprises in one container a pharmaceutical composition comprising a mercapto-acylamino acid and in a second container a pharmaceutical composition comprising an angiotensin converting enzyme inhibitor.

19. Process for the preparation of a compound of formula I as defined in claim 1, wherein the compound is prepared by an appropriate process selected from the following processes A, B and C, and wherein R, $R_1$, $R_2$, $R_3$ and n are defined in claim 1, including suitable protection:

Process A for compounds of formula I wherein R is $-OR_{12}$, condensation of a 3-thiopropionic acid of formula II, or a reactive derivative, with an amino acid ester of the formula III

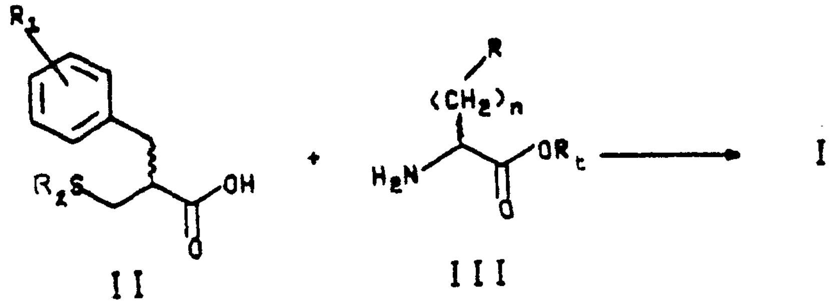

II III

wherein $R_t$ is defined in claim 1;

Process B for compounds of formula I wherein $R_3$ is $-NR_{12}R_{13}$, condensation of a 3-thiopropionic acid of formula II, or a reactive derivative, with an amino acid amide of formula V

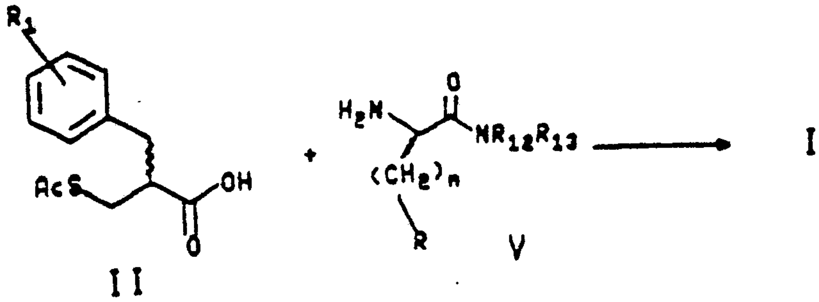

II V

wherein $R_{12}$ and $R_{13}$ are defined in claim 1;

Process C for compounds of formula I where $R_3$ is $-NR_{12}R_{13}$, condensation of a compound of formula Ia wherein $R_2$ is acetyl and $R_3$ is OH with an amine compound $NHr_{12}R_{13}$

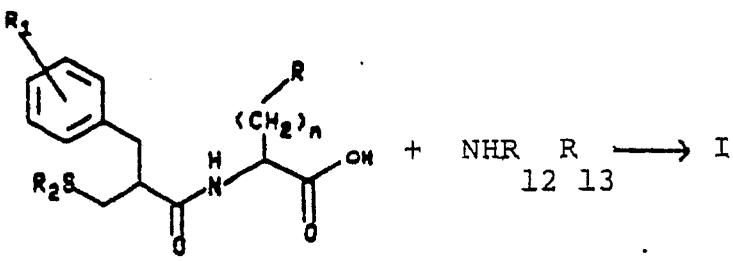

Ia

wherein $R_{12}$ and $R_{13}$ are defined in claim 1;

wherein Process A, Process B and Process C are followed by isolating the preferred isomer if desired and removal of the protecting groups, if necessary, to yield the desired products, and if desired, preparing a salt thereof.

European Patent Office

# PARTIAL EUROPEAN SEARCH REPORT

which under Rule 45 of the European Patent Convention shall be considered, for the purposes of subsequent proceedings, as the European search report

Application number
EP 89 11 5439

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US-A-4 401 677 (R. GREENBERG et al.) | | C 07 C 323/60<br>C 07 D 339/08<br>A 61 K 31/195 |
| A | EP-A-0 066 956 (PFIZER INC.) | | |
| | -------- | | |

TECHNICAL FIELDS SEARCHED (Int. Cl.4)

C 07 C 323/00
C 07 D 339/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-12,14-16,18,19

Claims searched incompletely:

Claims not searched: 13,17

Reason for the limitation of the search:

Method for treatment of the human or animal body by surgery or therapy (See art. 52(4) of the European Patent Convention)

| Place of search | Date of completion of the search | Examiner |
|---|---|---|
| The Hague | 10-10-1989 | ZAROKOSTAS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82